# EUROPÄISCHE PATENTSCHRIFT

(11) **EP 1 030 649 B1**
(45) Veröffentlichungstag und Bekanntmachung des Hinweises auf die Patenterteilung: **11.12.2002**
(21) Anmeldenummer: 98954471.3
(22) Anmeldetag: 31.10.1998
(51) Int. Cl.: A61K 7/42

(54) **LICHTSTABILE KOSMETISCHE FORMULIERUNG ENTHALTEND BUTYLMETHOXYDIBENZOYLMETHAN**
LIGHT STABLE COSMETIC FORMULATION CONTAINING BUTYLMETHOXYDIBENZOYLMETHANE
FORMULATION COSMETIQUE PHOTOSTABLE CONTENANT DU BUTYLMETHOXYDIBENZOYLMETHANE

(30) Priorität: 12.11.1997 DE 19750030
(43) Veröffentlichungstag der Anmeldung: 30.08.2000
(73) Patentinhaber: MERCK PATENT GmbH, 64293 Darmstadt (DE)
(72) Erfinder: KURZ, Thekla, D-64285 Darmstadt (DE); WILLE, Dorothee, D-64291 Darmstadt (DE); HITZEL, Sabine, D-64409 Messel (DE)
(86) Internationale Anmeldenummer: EP9806902
(87) Internationale Veröffentlichungsnummer: WO99024000

(56) Entgegenhaltungen:
- EP-A- 0 193 387
- EP-A- 0 610 926
- EP-A- 0 898 955
- WO-A-98/01107
- WO-A-98/13016
- DE-A- 4 227 806

## Beschreibung

Die vorliegende Erfindung betrifft unter anderem die Verwendung von im UVA-Bereich absorbierenden, nichtlöslichen anorganischen Partikeln zur Verbesserung der Photostabilität des organischen Lichtschutzfilters Butylmethoxydibenzoylmethan in kosmetischen Formulierungen.

Bekanntlich reagiert die Haut empfindlich auf Sonnenstrahlen, welche einen gewöhnlichen Sonnenbrand oder ein Erythem, aber auch mehr oder weniger ausgeprägte Verbrennungen hervorrufen können.

Sonnenstrahlen haben aber auch andere negative Wirkungen: sie bewirken, daß die Haut ihre Elastizität verliert und sich Falten bilden und führen somit zu einer frühzeitigen Alterung. Manchmal kann man auch Dermatosen beobachten. Im extremen Fall kommt es bei manchen Menschen zum Auftreten von Hautkrebs.

Bekanntlich wird der gefährlichste Teil der Sonnenstrahlen von den ultravioletten Strahlen mit einer Wellenläge von weniger als 400 nm gebildet. Bekannt ist auch, daß durch das Vorhandensein der Ozonschicht der Erdatmosphäre, die einen Teil der Sonnenstrahlung absorbiert, die untere Grenze der ultravioletten Strahlen, welche die Erdoberfläche erreichen, bei ca. 280 nm liegt.

Das Problem bei vielen Sonnenschutzpräparaten ist jedoch, daß viele Lichtschutzfilter empfindliche Substanzen sind und eine geringe Stabilität gegenüber den ultravioletten Strahlen aufweisen; sie zersetzen sich mehr oder weniger schnell. Dadurch ergeben sich Probleme bei der Haltbarkeit bzw. Wirksamkeit der entsprechenden kosmetischen Formulierungen. Diese Präparate zeigen keine dauerhafte Wirkung bei einer längeren Sonnenbestrahlung, so daß eine in regelmäßigen Abständen zu wiederholende Anwendung auf die Haut notwendig wird.

Die heute üblichen Sonnenschutzfilter in der kosmetik werden in UVA- bzw. UVB-Filter unterteilt. Für beide UV-Bereiche gibt es viele aus der Fachliteratur bekannte und bewährte Substanzen, beispielsweise seien hier nur Substanzen wie Eusolex ® 6300 oder Eusolex® 232, Dibenzoylmethane wie Eusolex® 9020 oder Eusolex® 8020, Benzophenone oder auch Octyl Triazone (Uvinul® T 150) aufgeführt.

Butylmethoxydibenzoylmethan (BMDM, Eusolex® 9020) ist ein häufig eingesetzter UVA-Filter, der jedoch stark durch UV-Bestrahlung abgebaut wird. Wird diese Substanz mit anderen organischen UVA-Filtem in einer Formulierung verarbeitet, erhöht sich die Stabilität des BMDM nicht.
Es besteht daher noch ein Mangel an Substanzen, die den Formulierungen zur Stabilisierung des Lichtschutzfilters BMDM zugegeben werden können, aber die ansonsten die Wirkung und die Zusammensetzung der kosmetischen Präparate nicht negativ beeinträchtigen.

Es erscheint somit wünschenswert, in Bezug auf die Lichtschutzfilter stabilere kosmetische Präparate zur Verfügung zu stellen, die mindestens den Lichtschutzfilter Butylmethoxydibenzoylmethan enthalten, die vor allem die UVA-Strahlen mit einer Wellenlänge zwischen 320 und 400 nm, welche die Haut bräunen, aber auch altem lassen, die Auslösung einer erythematösen Reaktion begünstigen oder diese Reaktion bei bestimmten Menschen vergrößern oder sogar phototoxische oder photoallergische Reaktionen auslösen können, absorbieren können, die aber auch, falls gewünscht, die UVB-Strahlen mit einer Wellenlänge zwischen 280 und 320 nm, die bei der Bildung eines Sonnenerythems eine entscheidende Rolle spielen, absorbieren können.

DE 42 27 806 A beschreibt kosmetische Zubereitungen zur Aufhellung der Haut, die eine Kombination von Butylmethoxydibenzoylmethan und Titandioxid enhalten.

WO 98/13016 A beschreibt Zusammensetzungen, die Butylmethoxydibenzoylmethan und oberflächenbehandeltes Zinkoxid enthalten.

EP-A-0 610 926 offenbart ein Sonnengel, welches Butylmethoxydibenzoylmethan und mikrofeines Titandioxid enthält.

EP-A-0 193 387 offenbart kosmetische Zusammensetzungen, die eine Kombination von Butylmethoxydibenzoylmethan und Titandioxid enthalten.

WO 98/01107 A beschreibt Zusammensetzungen, die Butylmethoxydibenzoylmethan und mikrofeines Titandioxid enthalten.

EP-A-0 898 955 offenbart kosmetische Zusammensetzungen, die beliebig ausgewählte organische und anorganische Lichtschutzfilter enthalten können.

Überraschend wurde nun gefunden, daß die Photostabilität von Butylmethoxydibenzoylmethan erhöht wird, wenn nichtlösliche anorganische Partikel, die im UVA-Bereich eine gute Absorption haben, zugesetzt werden.

Diese Substanzen bieten den großen Vorteil, keine toxischen oder allergischen Reakionen gegenüber der Haut zu zeigen.

Die Wirksamkeit der anorganischen UVA-Filter ist darauf zurückzuführen, daß sie als nichtlösliche Substanzen die Haut nicht penetrieren können, und dadurch in einer Schicht auf der Haut liegen bleiben. BMDM diffundiert als organisches Molekül in die obersten Schichten der Hornhaut, so daß aus einer aufgetragenen Formulierung ein Zweischichtensystem entsteht. Die untere Schicht, also das Butylmethoxydibenzoylmethan, erreichen daher nur noch geringere Energiemengen, was zu einem vermindertem Abbau des organischen Lichtschutzfilters führt.

Dieser Effekt der Penetration kann durch den Einsatz von Penetrationsförderern wie z. B. Dimethylsulfoxid oder Diethyltoluamid noch verstärkt werden.

Gegenstand der Erfindung ist die Verwendung von im UVA-Bereich absorbierenden, nichtlöslichen anorganischen, mikrofeinen Partikeln ausgewählt aus Titandioxid-Partikeln, die mit Cer- oder Eisenionen dotiert sind, zur Verbesserung der Photostabilität des Lichtschutzfilters Butylmethoxydibenzoylmethan in kosmetischen Formulierungen.

Zu diesen Partikeln zählt in erster Linie mikrofeines mit Cer- oder Eisenionen dotiertes Titandioxid, das in der Rutilmodifikation vorliegt, aber auch als Anatas vorliegen kann. Die Korngröße dieser Partikel liegt im Bereich von 10 nm bis 100 µm, vorzugsweise im Bereich von 10 nm bis 10 µm.

In einer ganz besonders bevorzugten Ausführungsform dieser Erfindung wird Titandioxid-Mikrorutil mit einer Korngröße bis zu 10 µm eingesetzt. Dieses Titanioxid-Mikrorutil ist unter dem Namen Eusolex® T-2000 bekannt und im Handel erhältlich (Fa. Merck KGaA, Darmstadt). Das mikrofeine Titandioxid in Rutilform besitzt eine sehr gute UVA-Absorption, und ist für die erfindungsgemäße Photostabilisierung des BMDM besonders gut geeignet.

Gegenstand der Erfindung ist auch eine kosmetische Zubereitung enthaltend den Lichtschutzfilter Butylmethoxydibenzoylmethan, dadurch gekennzeichnet, daß zur Verbesserung der Photostabilität dieses Lichtschutzfilters nichtlösliche anorganische, mikrofeine Partikel ausgewählt aus Titandioxid-Partikeln, die mit Cer- oder Eisenionen dotiert sind, die im UVA-Bereich eine gute Absorption aufweisen, zugegeben werden.

Der Gehalt an Butylmethoxydibenzoylmethan in der erfindungsgemäßen kosmetischen Zubereitung beträgt vorzugsweise 0,1 bis 10 Gew. %, insbesondere bevorzugt 0,5 bis 3 Gew. %.

Der Gehalt an anorganischen Partikeln beträgt 0,1 bis 30 Gew. %, insbesondere bevorzugt 0,5 bis 10 Gew. %.

Der Lichtschutzfilter Butylmethoxydibenzoylmethan kann in den erfindungsgemäßen kosmetischen Formulierungen alleine oder in Kombination mit einem oder mehreren UV-Filtern anderer Substanzklassen vorliegen, die jeweils in einer Menge von 0.01 bis 40 Gew. %, vorzugsweise von 0.1 bis 10 Gew. % enthalten sein können.

Die entsprechenden mikrofeinen Pigmente sind bekannt und können im Handel bezogen werden, z.B. von der Firma Merck KGaA, Darmstadt.

Sie zeigen so hervorragende Eigenschaften wie eine sehr gute Hautund Schleimhautverträglichkeit ohne toxische, allergisierende oder sensibilisierende Eigenschaften und keine Hautpenetration.

Ferner weist die Substanz eine hohe chemische Stabilität, d.h. keine Hydrolysierbarkeit, keine Photooxidierbarkeit, keine Oxidierbarkeit, hohe Thermostabilität und hohe Schweißfestigkeit auf.

Weiterhin ist diese Substanz mit den gängigen kosmetischen und pharmazeutischen Formulierungsgrundlagen gut verträglich und mischbar.

Gegenstand der Erfindung ist ferner die Verwendung von im UVA-Bereich absorbierenden, nichtlöslichen, anorganischen, mikrofeinen, mit Ger- oder Eisenionen dotierten Titandioxid-Partikeln zur Herstellung von kosmetischen Zubereitungen gemäß der Ansprüche.

Die erfindungsgemäßen Formulierungen können zum Schutz der Haut vor Sonnenstrahlen, zur vorbeugenden Behandlung von Entzündungen und Allergien der Haut, und zur Verhütung bestimmter Krebsarten verwendet werden.

In einer bevorzugten Ausführungsform dieser Erfindung liegt die kosmetische Formulierung in Form eines Sets aus zwei getrennten Formulierungen vor, dessen eine Formulierung, welche zuerst aufgetragen wird, Butylmethoxydibenzoylmethan und dessen zweite Formulierung, welche anschließend aufgetragen wird, die anorganischen Partikel enthält. Dabei entsteht sofort das gewünschte Zweischichtensystem.

Wird die erfindungsgemäße kosmetische Zubereitung zum Schutz menschlicher Epidermis gegen UV-Strahlen verwendet, liegt sie in verschiedenen, für diesen Typ üblicherweise verwendeten Formen vor. So kann sie insbesondere in Form öliger, ölig-wäßriger, wäßrigalkoholischer oder ölig-alkoholischer Lotionen, Emulsionen, wie als Creme oder als Milch (W/O oder O/W, wobei W = Wasser und O = Oil bedeutet), in Form ölig-alkoholischer, ölig-wäßriger oder wäßrigalkoholischer Gele oder als feste Stifte oder Puder vorliegen oder als Spray oder Aerosol konfektioniert sein.

Die erfindungsgemäße Formulierung kann weitere kosmetische Adjuvanzien enthalten, welche in dieser Art von Zubereitungen üblicherweise verwendet werden, wie z.B. Verdickungsmittel, weichmachende Mittel, Befeuchtungsmittel, grenzflächenaktive Mittel, Konservierungsmittel, Mittel gegen Schaumbildung, Parfüms, Wachse, Lanolin, Treibmittel, Farbstoffe und/oder Pigmente, welche das Mittel selbst. oder die Haut färben, und andere in der Kosmetik gewöhnlich verwendete Ingredienzien.

Man kann als Solubilisierungsmittel ein Öl, Wachs oder sonstigen Fettkörper, einen niedrigen Monoalkohol oder ein niedriges Polyol oder Mischungen davon verwenden. Zu den besonders bevorzugten Monoalkoholen oder Polyolen zählen Ethanol, i-Propanol, Propylenglycol, Glycerin und Sorbit.

Eine bevorzugte Ausführungsform der Erfindung ist eine Emulsion, welche als Schutzcreme oder -milch vorliegt und außer den organischen Lichtschutzfaktoren und den anorganischen UVA-Filtem Fettalkohole, Fettsäureester, insbesondere Triglyceride von Fettsäuren, Fettsäuren, Lanolin, natürliche oder synthetische Öle oder Wachse und Emulgatoren in Anwesenheit von Wasser umfaßt.

Weitere bevorzugte Ausführungsformen stellen ölige Lotionen auf Basis von natürlichen oder synthetischen Ölen und Wachsen, Lanolin, Fettsäureestem, insbesondere Triglyceriden von Fettsäuren, oder ölig-alkoholische Lotionen auf Basis eines Niedrigalkohols, wie Ethanol, oder eines Glycols, wie Propylenglykol, und/oder eines Polyols, wie Glycerin, und Ölen, Wachsen und Fettsäureestern, wie Triglyceriden von Fettsäuren, dar.

Die erfindungsgemäße kosmetische Zubereitung kann auch als alkoholisches Gel vorliegen, welches einen oder mehrere Niedrigalkohole oder -polyole, wie Ethanol, Propylenglycol oder Glycerin, und ein Verdickungsmittel, wie Kieselerde umfaßt. Die ölig-alkoholischen Gele enthalten außerdem natürliches oder synthetisches Öl oder Wachs.

Die festen Stifte bestehen aus natürlichen oder synthetischen Wachsen und Ölen, Fettalkoholen, Fettsäuren, Fettsäureestern, Lanolin und anderen Fettkörpern.

Ist eine Zubereitung als Aerosol konfektioniert, verwendet man in der Regel die üblichen Treibmittel, wie Alkane, Fluoralkane und Chlorfluoralkane.

Ist eine Formulierung als Spray konfektioniert, verwendet man in der Regel wäßrig-alkoholische Lösungen.

Die erfindungsgemäßen kosmetischen Zubereitungen können mit Hilfe von Techniken hergestellt werden, die dem Fachmann wohl bekannt sind.

Auch ohne weitere Ausführungen wird davon ausgegangen, daß ein Fachmann die obige Beschreibung in weitesten Umfang nutzen kann.

Die folgenden Beispiele sollen dazu dienen, die Erfindung näher zu erläutern.

### Beispiel 1

Aus folgenden Komponenten stellt man eine erfindungsgemäße Sonnenschutzcreme (O/W) her.

| | | | Gew. % |
|---|---|---|---|
| A | Eusolex® 9020 (Art.-Nr. 105844) (Butylmethoxydibenzoylmethan) | (1) | 1.00 |
| | Eusolex® T-2000 (Art.-Nr. 105373) | (1) | 5.00 |
| | Arlatone® 983 S | (2) | 1.50 |
| | Arlatone® 985 | (2) | 2.20 |
| | Brij® 76 | (2) | 1.50 |
| | Miglyol® 812 Neutralöl | (3) | 10.00 |
| | | | |
| B | Sorbitol F flüssig (Art.-Nr. 102993) | (1) | 2.50 |
| | Propandiol-1,2 (Art.-Nr. 107478) | (1) | 2.50 |
| | Konservierungsstoffe | (1) | q.s. |
| | demin. Wasser | | ad 100.00 |
| | | | |
| C | Carbomer 934 | (4) | 0.50 |
| | | | |
| D | Tris(hydroxymethyl)aminomethan | | |
| | (Art.-Nr. 108386) | (1) | 0.36 |
| | demin. Wasser | | 9.64 |

### Herstellung:

Man mischt Phase B, gibt das Carbomer 934 hinzu und läßt die Mischung aufquellen, bis sie homogen ist. Danach gibt man die vorgemischte Phase D hinzu und homogenisiert. Man erwärmt nun auf 80 °C. Die Phase A wird vereinigt und auf 75 °C erwärmt. Unter langsamen Rühren wird Phase A in die vorher aus B-D hergestellte Phase eingerührt.

### Konservierungsstoffe:

0.05% Propyl-4-hydroxybenzoat (Art.-Nr. 107427)
0.15% Methyl-4-hydroxybenzoat (Art.-Nr. 106757)

### Bezugsquellen:

Merck KGaA, Darmstadt
ICI, Essen
Hüls Troisdorf AG, Witten
Goodrich, Neuss

### Beispiel 2

Aus folgenden Komponenten stellt man eine erfindungsgemäße Sonnenschutzcreme (O/W) aus zwei getrennten Formulierungen her.

| Formulierung I | | | |
|---|---|---|---|
| | | | Gew.% |
| A | Eusolex® T-2000 (Art.-Nr. 105373) | (1) | 5.00 |
| | Arlatone® 983 S | (2) | 1.50 |
| | Arlatone® 985 | (2) | 2.20 |
| | Brij® 76 | (2) | 1.50 |
| | Miglyol® 812 Neutralöl | (3) | 10.00 |
| | | | |
| B | Sorbitol F flüssig (Art.-Nr. 102993) | (1) | 2.50 |
| | Propandiol-1,2 (Art.-Nr. 107478) | (1) | 2.50 |
| | Konservierungsstoffe | (1) | q.s. |
| | demin. Wasser | | ad 100.00 |
| | | | |
| C | Carbomer 934 | (4) | 0.50 |
| | | | |
| D | Tris(hydroxymethyl)aminomethan | | |
| | (Art.-Nr. 108386) | (1) | 0.36 |
| | demin. Wasser | | 9.64 |

### Herstellung:

Man mischt Phase B, gibt das Carbomer 934 hinzu und lässt die Mischung aufquellen, bis sie homogen ist. Danach gibt man die vorgemischte Phase D hinzu und homogenisiert. Man erwärmt nun auf 80 °C. Die Phase A wird vereinigt und auf 75 °C erwärmt. Unter langsamen Rühren wird Phase A in die vorher aus B-D hergestellte Phase eingerührt.

### Konservierungsstoffe:

0.05% Propyl-4-hydroxybenzoat (Art.-Nr. 107427)
0.15% Methyl-4-hydroxybenzoat (Art.-Nr. 106757)

| Formulierung II | | | |
|---|---|---|---|
| | | | Gew. % |
| A | Eusolex® 9020 (Art.-Nr. 105844) | (1) | 5.00 |
| | Arlatone® 983 S | (2) | 1.50 |
| | Arlatone® 985 | (2) | 2.20 |
| | Brij® 76 | (2) | 1.50 |
| | Miglyol® 812 Neutralöl | (3) | 10.00 |
| | | | |
| B | Sorbitol F flüssig (Art.-Nr. 102993) | (1) | 2.50 |
| | Propandiol-1,2 (Art.-Nr. 107478) | (1) | 2.50 |
| | Konservierungsstoffe | (1) | q.s. |
| | demin. Wasser | | ad 100.00 |
| | | | |
| C | Carbomer 934 | (4) | 0.50 |
| | | | |
| D | Tris(hydroxymethyl)aminomethan | | |
| | (Art.-Nr. 108386) | (1) | 0.36 |
| | demin. Wasser | | 9.64 |

### Herstellung:

Man mischt Phase B, gibt das Carbomer 934 hinzu und lässt die Mischung aufquellen, bis sie homogen ist. Danach gibt man die vorgemischte Phase D hinzu und homogenisiert. Man erwärmt nun auf 80 °C. Die Phase A wird vereinigt und auf 75 °C erwärmt. Unter langsamen Rühren wird Phase A in die vorher aus B-D hergestellte Phase eingerührt.

### Konservierungsstoffe:

0.05% Propyl-4-hydroxybenzoat (Art.-Nr. 107427)
0.15% Methyl-4-hydroxybenzoat (Art.-Nr. 106757)

### Bezugsquellen:

Merck KGaA, Darmstadt
ICI, Essen
Hüls Troisdorf AG, Witten
Goodrich, Neuss

Die beiden Formulierungen werden separat abgefüllt. Bei der Anwendung wird zuerst Formulierung I, danach Formulierung II auf die Haut aufgetragen.

## Patentansprüche

1. Kosmetische Zubereitung enthaltend Butylmethoxydibenzoylmethan als Lichtschutzfilter, **dadurch gekennzeichnet, daß** darin zur Verbesserung der Photostabilität im UVA-Bereich absorbierende, nichtlösliche anorganische, mikrofeine Partikel ausgewählt aus Titandioxid-Partikeln, die mit Cer- oder Eisenionen dotiert sind, enthalten sind, die eine Korngröße von 10 nm bis 100 µm besitzen.

2. Kosmetische Zubereitung nach Anspruch 1, **dadurch gekennzeichnet, daß** darin als nichtlösliche anorganische Partikel Titandioxid-Mikrorutil mit einer Korngröße bis zu 10 µm enthalten sind.

3. Kosmetische Zubereitung nach einem oder mehreren der Ansprüche 1 bis 2, **dadurch gekennzeichnet, daß** der Gehalt an anorganischen Partikeln in der kosmetischen Zubereitung 0,1 bis 30 Gew. % beträgt.

4. Kosmetische Zubereitung nach einem der Ansprüche 1 bis 3, **dadurch gekennzeichnet, daß** der Gehalt an Butylmethoxydibenzoylmethan in der kosmetischen Zubereitung 0,1 bis 10 Gew. % beträgt.

5. Kosmetische Zubereitung nach einem der Ansprüche 1 bis 4, **dadurch gekennzeichnet, daß** sie aus einem Set von zwei getrennt vorliegenden Formulierungen besteht, dessen eine Formulierung Butylmethoxydibenzoylmethan und dessen zweite Formulierung die anorganische Partikel enthält.

6. Verwendung von im UVA-Bereich absorbierenden, nichtlöslichen, anorganischen, mikrofeinen Partikeln ausgewählt aus Titandioxid-Partikeln, die mit Ceroder Eisenionen dotiert sind und die eine Korngröße von 10 nm bis 100 µm besitzen zur Verbesserung der Photostabilität des Lichtschutzfilters Butylmethoxyldibenzoylmethan in kosmetischen Formulierungen

7. Verwendung von im UVA-Bereich absorbierenden, nichtlöslichen, anorganischen, mikrofeinen Partikeln gemäß Anspruch 6 zur Herstellung von kosmetischen Zubereitungen gemäß der Ansprüche 1-5.

## Claims

1. Cosmetic preparation comprising butylmethoxydibenzoylmethane as light protection filter, **characterized in that** insoluble inorganic microfine particles which absorb in the UVA region, chosen from titanium dioxide particles, which have been doped with cerium ions or iron ions and which have a particle size of from 10 nm to 100 µm are present therein to improve the photostability.

2. Cosmetic preparation according to Claim 1, **characterized in that** the insoluble inorganic particles present therein are titanium dioxide microrutile having a particle size up to 10 µm.

3. Cosmetic preparation according to one or more of Claims 1 to 2, **characterized in that** the content of inorganic particles in the cosmetic preparation is from 0.1 to 30% by weight.

4. Cosmetic preparation according to one of Claims 1 to 3, **characterized in that** the content of butylmethoxydibenzoylmethane in the cosmetic preparation is from 0.1 to 10% by weight.

5. Cosmetic preparation according to one of Claims 1 to 4, **characterized in that** it consists of a set of two separate formulations, one formulation of which comprises butylmethoxydibenzoylmethane, and the second formulation of which comprises the inorganic particles.

6. Use of insoluble inorganic microfine particles which absorb in the UVA region, chosen from titanium dioxide particles which have been doped with cerium ions or iron ions, and which have a particle size of from 10 nm to 100 µm, for improving the photostability of the light protection filter butylmethoxydibenzoylmethane in cosmetic formulations.

7. Use of insoluble inorganic microfine particles which absorb in the UVA region according to Claim 6 for the preparation of cosmetic preparations according to Claims 1 - 5.

## Revendications

1. Préparation cosmétique contenant du butylméthoxydibenzoylméthane comme filtre de protection contre la lumière, **caractérisée en ce qu'**elle contient pour améliorer la photostabilité dans la région A ultraviolette des particules inorganiques microfines, non solubles, absorbantes, choisies parmi les particules de dioxyde de titane dopées avec des ions cérium ou des ions fer, ayant une dimension de grain comprise entre 10 nm et 100 µm.

2. Préparation cosmétique selon la revendication 1, **caractérisée en ce qu'**elle contient, comme particules inorganiques non solubles, du dioxyde de titane microrutile ayant une dimension de grain allant jusqu'à 10 µm.

3. Préparation cosmétique selon l'une des revendications 1 et 2, **caractérisée en ce que** la concentration en particules inorganiques dans la préparation cosmétique est comprise entre 0,1 % et 30 % en poids.

4. Préparation cosmétique selon l'une des revendications 1 à 3, **caractérisée en ce que** la concentration en butylméthoxydibenzoylméthane dans la préparation cosmétique est comprise entre 0,1 et 10 % en poids.

5. Préparation cosmétique selon l'une des revendications 1 à 4, **caractérisée en ce qu'**elle est constituée de set de deux formulations se présentant, de façon séparée, une formulation contenant le butylméthoxydibenzoylméthane et l'autre formulation des particules inorganiques.

6. Utilisation des particules inorganiques microfines, non solubles, absorbantes dans la région A ultraviolette, choisies parmi les particules de dioxyde de titane dopées avec des ions cérium ou des ions fer et présentant une dimension de grain comprise entre 10 nm et 100 µm, pour améliorer la photostabilité du filtre de protection contre la lumière butylméthoxydibenzoylméthane dans les formulations cosmétiques.

7. Utilisation des particules inorganiques microfines, non solubles, absorbantes dans la région A ultraviolette, selon la revendication 6, pour la fabrication de préparations cosmétiques selon les revendications 1 à 5.
